# EUROPEAN PATENT SPECIFICATION

(11) **EP 1 244 667 B1**
(45) Date of publication and mention of the grant of the patent: **05.04.2006**
(21) Application number: 00990110.9
(22) Date of filing: 27.12.2000
(51) Int. Cl.: C07D 473/34, A61K 31/70, C07H 21/00

(54) **CYCLOHEXENE NUCLEIC ACIDS**
CYCLOHEXENNUKLEINSÄUREN
ACIDES NUCLEIQUES CONTENANT CYCLOHEXENE

(30) Priority: 30.12.1999 US 173728 P
(43) Date of publication of application: 02.10.2002
(73) Proprietor: K.U. LEUVEN RESEARCH & DEVELOPMENT, 3000 Leuven (BE)
(72) Inventor: WANG, Jing, B-9100 Sint-Niklaas (BE); HERDEWIJN, Piet, B-3111 Rotselaar (BE)
(74) Representative: Bird, Ariane
(86) International application number: PCT/IB2000/002041
(87) International publication number: WO 2001/049687

(56) References cited:
- DE-A- 19 815 901
- US-A- 5 034 506
- HERDEWIJN P: "Targeting RNA with Conformationally Restricted Oligonucleotides" LIEBIGS ANNALEN: ORGANIC AND BIOORGANIC CHEMISTRY, VCH PUBLISHERS, US, no. 9, 1 September 1996 (1996-09-01), pages 1337-1348, XP002094305 ISSN: 0947-3440
- WANG J ET AL: "Enantioselective synthesis and conformational study of cyclohexene carbocyclic nucleosides" JOURNAL OF ORGANIC CHEMISTRY, AMERICAN CHEMICAL SOCIETY. EASTON, US, vol. 64, no. 21, 15 October 1999 (1999-10-15), pages 7820-7827, XP002157746 ISSN: 0022-3263 cited in the application
- HERDEWIJN, P. ET AL.: "Cyclohexene nucleic acids (CeNA): Serum stable oligonucleotides that activate RNase H and increase duplex stability with complementary RNA" J. AM. CHEM. SOC., vol. 122, 2000, pages 8595-8602, XP002179475

## Description

### Field of the Invention

This patent application relates to cyclohexene-containing oligonucleotides. Such compounds have been found to exhibit nuclease resistance, activate RNase H, and hybridize with complementary RNA or DNA.

### Background of the Invention

Replacement of the five membered furanose ring of natural DNA and RNA with a six membered ring has been found to increase the conformational rigidity of resulting oligomers. From the structural point of view, such oligomers can be divided in three groups: (1) those with the base moiety positioned at the 1'-position (pyranose nucleic acids); (2) those with the base moiety positioned at the 2'-position (hexitol nucleic acids); and (c) nucleic; acids lacking a ring oxygen atom. It has been found that the known oligomers of the first type do not hybridize with natural nucleic acids, while those of the latter two types do.

### Summary of the Invention

In one aspect, the present invention provides oligomeric compounds comprising a plurality of covalently-bound nucleoside units that individually include a nucleosidic base and either a furanosyl moiety or a cyclohexenyl moiety covalently bound to the base. At least one of the units in these compounds includes a nucleosidic base covalently bound to a cyclohexenyl moiety. Preferred compounds according to the invention are those in which the nucleoside units are covalently bound such that the base portions thereof form a mixed base sequence that is complementary to a RNA base sequence or a DNA base sequence. The nucleoside units can include substituent groups (such as hydroxyl and/or methoxy groups) on any of their respective base portions, furanosyl moieties, and/or cyclohexenyl moieties. The units can be bound to one another using phosphodiester linkages, phosphorothioate linkages, or any a variety of internucleoside linkages known in the art.

In another aspect, the present invention provides methods that involve contacting a human or some other mammal with one or more of the above-noted compounds. In certain preferred embodiments, the mammal has or is suspected to have a disease associated with undesired protein production, and an actual reduction in protein production is thereafter detected.

The present invention also provides pharmaceutical composition. Preferred compositions comprise one of the compounds of the invention together with a pharmaceutically acceptable carrier or diluent. Compositions of the invention can exist, for example, in any of the physical forms (e.g., liquid, tablet, or powder) known to those skilled in the art.

In yet another aspect, the present invention provides methods that comprise contacting one of the above-noted compounds with RNA or DNA. Such contacting can be effected *in vitro* or *in vivo,* and preferably results in hybridization of the compound with the RNA or DNA.

### Brief Description of the Figures

Figure 1 shows a synthetic scheme for the phosphoramidite building block of 9-[(1S,4R,5S)-5-hydroxy-4-hydroxymethyl-2-cyclohexeneyl]adenine.
Figure 2 shows a conformational globe of puckered cyclohexene.
Figure 3 shows the equilibrium between two half-chair conformations of a cyclohexene nucleoside.
Figure 4 shows the structure of modified nucleosides with an adenine base moiety: cyclohexenyl A (A₁) and anhydrohexitol A (A₂).
Figure 5 shows UV mixing curves of (A₁)₁₃ and dT₁₃ carried out at pH 7.5 in 0.1 M NaCl.
Figure 6 shows CD spectra ofdsDNA before and after incorporation of A₁ and A₂ into one of the DNA strands.
Figure 7 shows CD spectra of DNA/RNA complexes before and after incorporation of A₁ and A₂ into one of the DNA strands.
Figure 8 shows CD spectra of dsRNA before and after incorporation of A₁ and A₂ into one of the DNA strands.
Figure 9 shows: (a) the sequence of the modified Dickerson dodecamer, which forms a symmetric duplex; (b) imino region of 1D WATERGATE NOESY (250 ms) spectra of the modified 12mer in 90% (H₂O/10% D₂O (10mM KH₂PO₄, 10 µM EDTA, pH 7.5); and (c) imino region of 2D WATERGATE NOESY (250 ms) spectra of the modified 12mer in 90% (H₂O/10% D₂O (10mM KH₂PO₄, 10 µM EDTA, pH 7.5). Assignments and connectivities (solid lines) are indicated for the imino resonances.
Figure 10 shows: (a) 1D H1' and H5 proton region of compound (I); (b) (H8/H6/H2) - H1'/H5 region of the 250 ms NOESY of compound (I) at 20 °C in 100% D₂O. Sequential H8/H6₍ₙ₎-H1'₍ₙ₎-H8/H6₍ₙ₊₁₎ connectivities starting from the 5' end (Cl) (labeled by arrow) are shown in solid lines (resonances at 7.42 and 7.61 ppm are from A5H2 and A6H2, respectively); and (c) H5-H6 region of the DQF COSY of compound (I) at 20 °C in 100% D₂O.

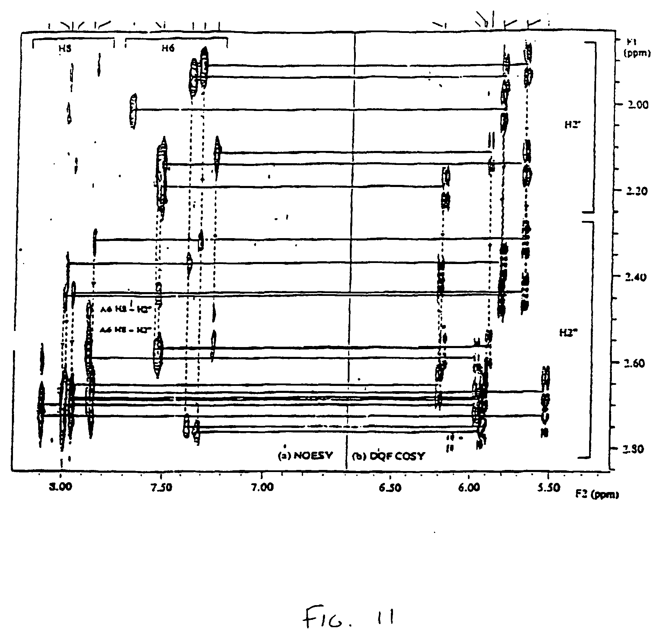

Figure 11 shows a NOESY-COSY connectivity diagram for sequential assignments via NOEs between base protons and the 2'C protons using NOESY with a mixing time of 50ms and DQF-COSY representing the region of the H1' - H2'/H2" crosspeaks. In the NOESY spectrum (a), successive cross peaks dₛ(2";6,8) and dᵢ(6,8; 2') are connected by dashed vertical lines. In the COSY spectrum (b), the crosspeaks H1' - H2' and H1' - H2" of each nucleotide are connected by dashed vertical lines. Horizontal lines connect the crosspeaks dᵢ(6,8; 2') with H1' - H2' crosspeaks of the same nucleotide and the H1 - H2" crosspeaks are connected with the crosspeaks dₛ(2"; 6,8). Following the arrows, the sequential assignment can be followed from C1 to G12. Sequence-specific assignments from the base protons and the H1' protons are indicated at the top. The crosspeaks indicated with the asterisk represent the H4' - H5'/H6/ crosspeaks.
Figure 12 shows double chimeric duplexes used to study RNase H activities (M: 2'-OMe; r: ribo; d: deoxy).
Figure 13 shows RNase H mediated hydrolysis of chimeric 2'-O-methyl RNA-RNA oligonucleotide. *lane* 1: RNA target; *lane* 2: partial alkaline hydrolysis (0.2 M Na₂CO₃, 80 °C, 5 min) of RNA target; *lanes* 3-5: RNase H activity towards DNA/RNA duplex (incubation times 1h, 3h, 6h); *lanes* 6-8: RNase H activity towards CeNA/RNA duplex (incubation times 1h, 3h, 6h); *lanes* 9-11: RNase H activity towards HNA/RNA duplex (incubation times 1h, 3h, 6h).
Figure 14 shows serum stability (50% Fetal calf serum in PBS) of oligoadenylates at 0, 5, 10, 30, 60, 180 min. incubation time at 37 °C. A. dA₁₃ B. oligo(A₁)₁₃ C. oligo(A₂)₁₃.

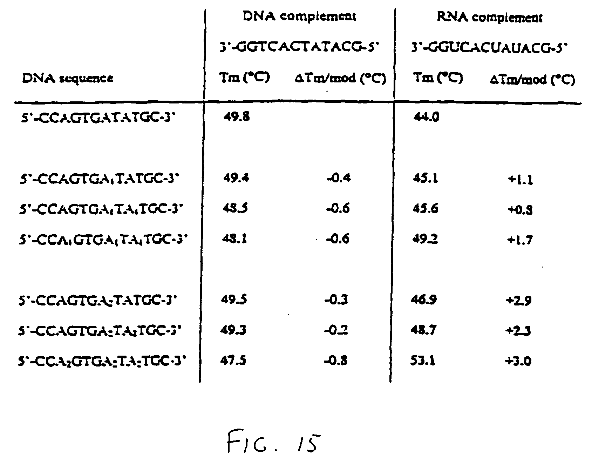

Figure 15 is a table showing the thermal stability of DNA/DNA and DNA/RNA duplexes after incorporation of A₁ and A₂.
Figure 16 is a table showing the influence of incorporation of A₁ on the stability of RNA/DNA and RNA/RNA duplexes.
Figure 17 is a table showing the thermal stability offully modified oligo(A₁)₁₃ with DNA and RNA complement.
Figure 18 is a table showing the chemical shifts (ppm) of the modified duplex (I) for the aromatic and anomeric protons compared with those of the Dickerson duplex (II).

### Detailed Description of the Invention

In pyranose nucleic acids with the nucleobase at the anomeric center, the nucleobase is equatorially oriented both at the level of the monomer and at the oligomeric level. These oligomers form strong self-complementary duplexes in case hybridization is not hindered for steric reasons. They do not hybridize with natural nucleic acids. In hexitol nucleic acids with a base in position 2, the nucleobases are axially oriented at the level of the monomer as well as at the level of the oligomer. Strong self-complementary hybridization and hybridization with natural nucleic acids is observed. The RNA-selective hybridization is explained by the similarities between the hexitol ring and a furanose ring frozen in its 3'-endo conformation. Pyranose nucleic acids have a 1,3-relationship between the nucleobase and the hydroxymethyl function. The hexitol-based nucleosides have a 1,4-relationship between both functionalities.

In cyclohexane nucleic acids (CNA), the conformation of the nucleoside is supposed to be different when analyzed as a monomer and after incorporation in an oligonucleotide. At the monomeric level the nucleobase is equatorially oriented, while at the oligomeric level the nucleobase is axially oriented. The axial orientation is preferred for hybridization of CNA with natural nucleic acids. The cyclohexane nucleic acids occupy a position between both other series (pyranoses and hexitols). A cyclohexane ring usually adopts a chair conformation and chair-chair interconversion is characterized by an activation barrier of ±12 kcal/mol. Introduction of a double bond into a cyclohexane ring gives a cyclohexene ring with a half-chair form as a global minimum.

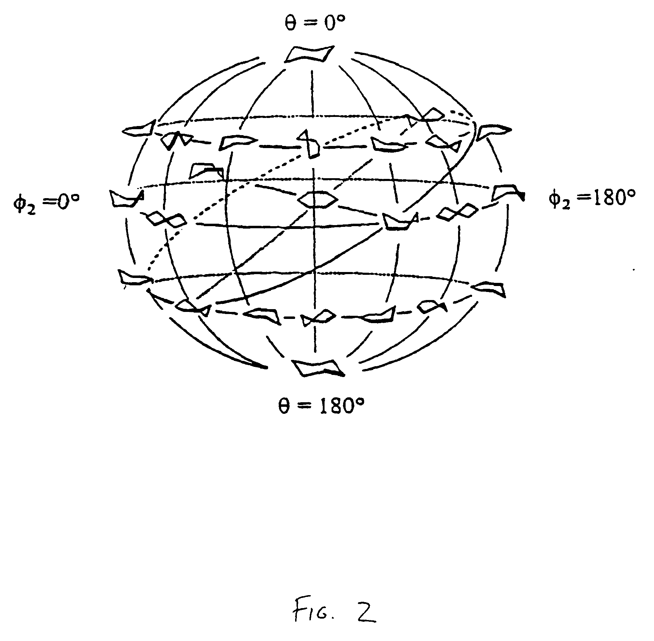

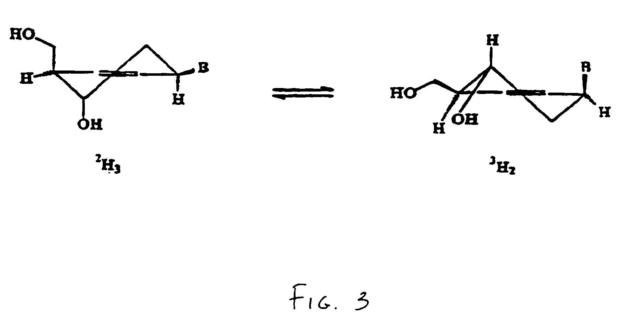

The conformational globe of a puckered cyclohexene is given in Figure 2. The chair forms are located at the poles. In the equator plane, boat and twist-boat forms alternate. The Tropic of Cancer at the northern hemisphere and the Tropic of Capricorn at the southern hemisphere are the locations of the envelope and half-chair forms. The half-chair forms are located at θ = 52.7°; ∅₂ = 210° and θ = 127.3°; ∅₂ = 30° and they interconvert via the boat forms, given along the indicated ellipsis. The energy barrier for half-chair-half-chair interconversion via the boat form is ± 5.5 kcal/mol. The cyclohexene system is more flexible than the cyclohexane system, and approaches more the flexibility of a furanose ring. When the two extreme half-chair conformations (³H₂ and ²H₃) are compared, it can be concluded that the cyclohexene ring in its ³H₂ form is a good mimic of a furanose ring in its C3'-endo conformation (Figure 3). Additionally, a cyclohexene ring in its ²H₃ conformation may be considered as a good mimic of a C2'-endo puckered furanose. We calculated that the energy difference between both conformations (for a purine nucleoside) is about 1.6 kJ/mol and that the half-chair conformation with the base in a pseudoaxial position (³H₂) is preferred. The low energy difference between both conformers can be explained by the stabilization of the ³H₂ form via π-σ* interactions, while the ²H₃ is sterically less demanding.

Because of this interesting equilibrium we started investigations on the incorporation of cyclohexene nucleosides in oligonucleotides and investigated: (a) whether or not the cyclohexene nucleoside can adopt different conformations when incorporated in a DNA or RNA chain; (b) whether or not the cyclohexene nucleoside can stabilize DNA and/or RNA duplexes; (c) whether or not CeNA is stable against nuclease degradation; and (d) whether or not the modified CeNA/RNA duplex is able to induce RNase H cleavage of the RNA strand. This last hypothesis is based on the fact that RNase H discriminates between dsRNA and RNA/DNA hybrids and recognizes the minor groove of the double stranded nucleic acids.

It is very likely that RNase H recognizes the three-dimensional structure of a RNA/DNA hybrid better than it does a double stranded RNA. The minor groove of a RNA/DNA, hybrid is narrower than of A-form duplexes (dsRNA) but wider than that of B-form (dsDNA) duplexes. Due to this narrower minor groove, it can be expected that RNase H is able to bind across the minor groove simultaneously contacting both the RNA and DNA strands while no close interactions could occur with a wide groove as in A-form dsRNA. Solution phase conformational analysis shows that the DNA/RNA duplex has an intermediate structure between the typical A- and B-form, but globally closer to the A-form than the B-form. The ribose moieties of the RNA strand have a N-conformation while the deoxyribose moieties of the DNA strand deviate from the typical S-conformation. It has been observed that the sugars of the DNA strand in a DNA/RNA duplex may adopt the 04'-endo conformation (P: 100 to 130°). Moreover, there is much more flexibility in the DNA/RNA hybrids than in the DNA duplex or RNA duplex. Therefore we hypothesized that the flexible cyclohexene system might be an excellent mimic of the natural furanose ring, and that CeNA/RNA duplex might activate RNase H.

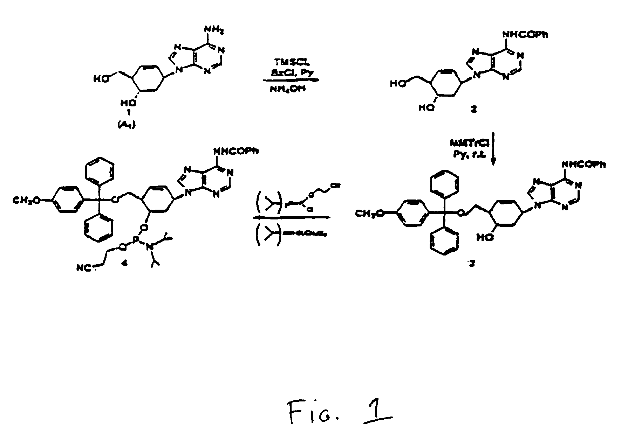

To investigate the properties of cyclohexene-containing nucleic acids, we first synthesized the protected phosphoramidite nucleoside with an adenine base moiety. The synthesis starts from R-(-)-carvone. The synthesis of the cyclohexene nucleoside itself is generally as set forth in Wang, *et al., J. Org. Chem.,* 1999, 64, 7820-7827. The adenine base moiety was protected with a benzoyl group and the primary hydroxyl group with a monomethoxytrityl group (Figure 1). The secondary hydroxyl group is reacted with 2-cyanoethyl-N,N-diisopropyl-chlorophosphoramidite to yield the nucleotide building block. Assembly of the monomers into oligonucleotides was done generally according to Maurinsh, *et al.,* P*. Chem. Eur. J.,* 1999, 5, 2139-2150. The excess of amidite used during coupling was collected, hydrolyzed to the H-phosphonate after which the phosphonate moiety was cleaved generally as described by Song, *et al., Tetrahedron Lett.,* 1999, 40, 4153-4156,allowing a second batch of amidite to be synthesized. This enabled synthesis of the large scale oligo (Dickerson duplex for NMR studies) and the oligo for RNase H studies. As a proof of correct incorporation of the cyclohexenyl building block, one of the synthesized oligonucleotides [5'-d(CGC GAA₁ TTC GCG-3')] was desalted by cation exchange beads and analyzed by mass spectrometry. The ESI-MS spectrum showed several peaks corresponding to multiply charged ions of the sample in the charge states (M - 3H]³⁻ to [M- 6H]⁶⁻. The deconvoluted spectrum, calculated by Max Ent I Processing, indicates the experimental monoisotopic mass to be 3654.71 Da, confirming the identity of the oligonucleotide (calculated 3654.74 Da). Further MS/MS analysis of the (M - 4H]⁴⁻ peak at 912.68 Da allowed several fragment peaks to be assigned.

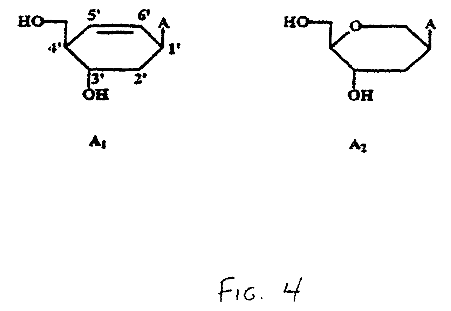

To test the influence of a cyclohexenyl nucleoside on the stability of dsDNA, DNA/RNA and dsRNA, sequences we incorporated cyclohexenyl nucleosides (Figure 4, A₁) in a mixed purine, pyrimidine DNA sequence (5'-CCAGTGATATGC-3') and in the same RNA sequence (5'CCAGUGAUAUGC-3'). The influence ofthe cyclohexenyl nuclooside on duplex stability was compared with that of an anhydrohexitol nucleoside (Figure 4, A). In both cases one, two and ΔTm/mod of -0.5oC was observed, independent whether 1, 2 or 3 modified nucleosides were incorporated. A similar drop in stability is found after incorporation of anhydrohexitolA (A₂). The influence of A₁ and A₂ on the duplex stability of dsDNA does not seem to be fundamentally different.

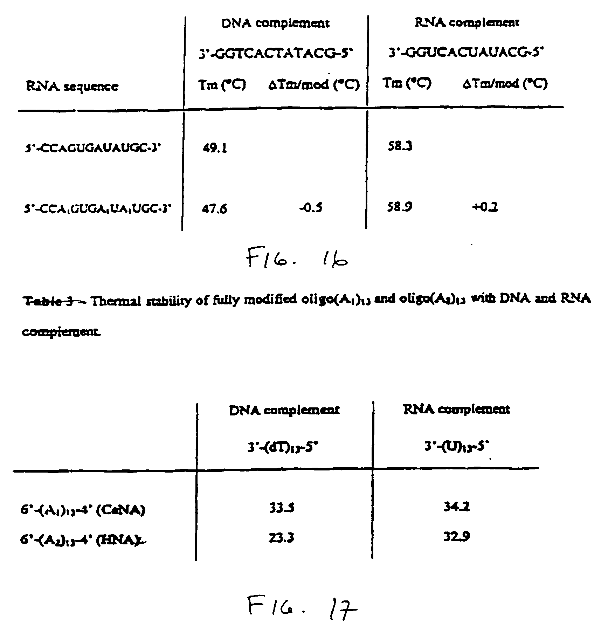

Of perhaps more interest is the influence of cyclohexenylA on the stability of DNA/RNA duplex. Incorporation of 1, 2 or 3 cyclohexenylA nucleosides in the DNA strand increase duplex stability with +1.1°C, +1.6°C and +5.2°C respectively, which means a ΔTm/mod of +1.1°C, +0.8°C and +1.7°C. As expected, the influence on duplex stability is dependent on the incorporation site of A₁. The stabilization effect of A₂ on the DNA/RNA duplex is twice as high as of A1, the sequence selective effect is the same. When cyclohexenylA is incorporated in the RNA strand of a DNA/RNA duplex, a ΔTM/mod of - 0.5°C is observed (Figure 16). It should, however, be noticed that the difference in stability of the DNA/RNA complex and the RNA/DNA complex is 5°C. Incorporation of three residues of A1 in a dsRNA sequence leads to an increase in duplex stability of+0.6°C. The increase in duplex stability after incorporation of A1, using RNA as the complementary strand, suggests that the conformation of A1 in duplex may be related to the 3'-endo conformation of the ribofuranose sugar. The ³H₂ conformation of A₁ is indeed the most stable conformation of cyclohexenylA. and conformational preorganization may be the basis of this duplex stabilization.

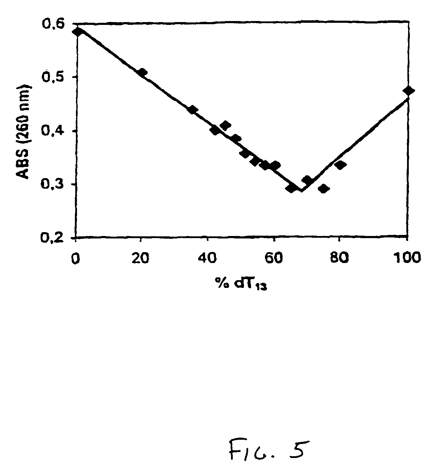

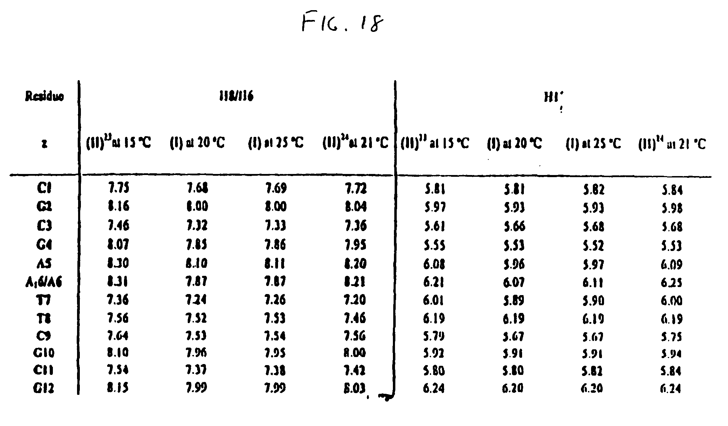

We compared the stability of the complexes formed between a cyclohexenyl oligoadenylate or an anhydrohexitol oligoadenylate and poly(dT) or poly(U) (Figure 17). The complex formed between cyclohexenyl oligoadenylate and poly(U) is of similar stability than between anhydrohexitol oligoadenylate and poly(U). However, of interest is the observation that the cyclohexenyl oligoadenylate-poly(dT) complex is much more stable (ΔTm: +10oC) than the corresponding anhydrohexitol oligoadenylate-poly(dT) complex. The nature of the cyclohexenyl oligoadenylate-poly(dT) complex was determined by a titration experiment (Figure 5) and found too be a triplex origin [(dT)13-(dT)13-(DT)13]. The complex formed between cyclohexenyl oligoadenylate and its DNA (poly(dT)) or RNA (poly(U)) complement is of similar stability. These results may indicate that CeNA are flexible molecules and that cyclohexene nucleotides can be accommodated in different kind of complexes.

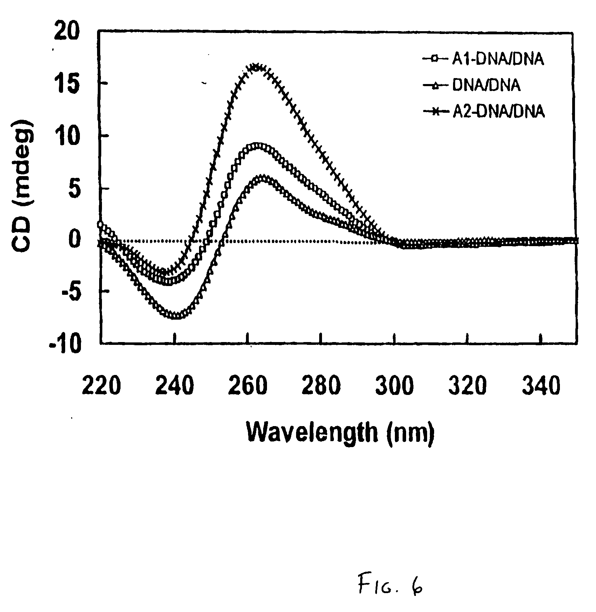

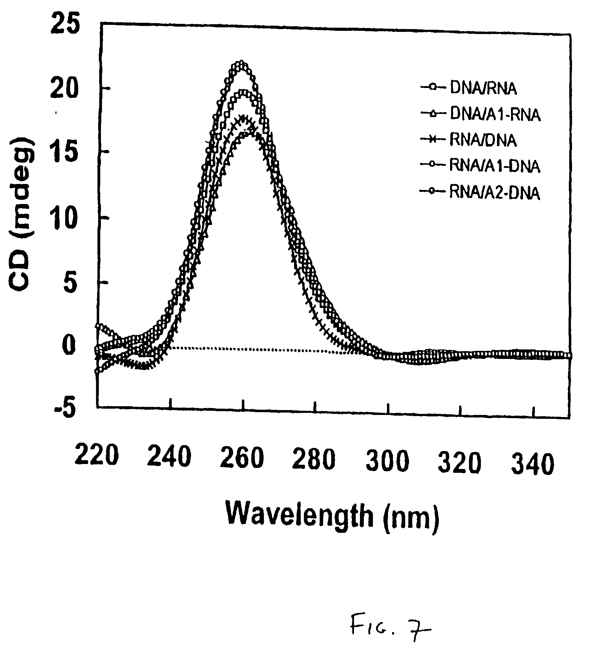

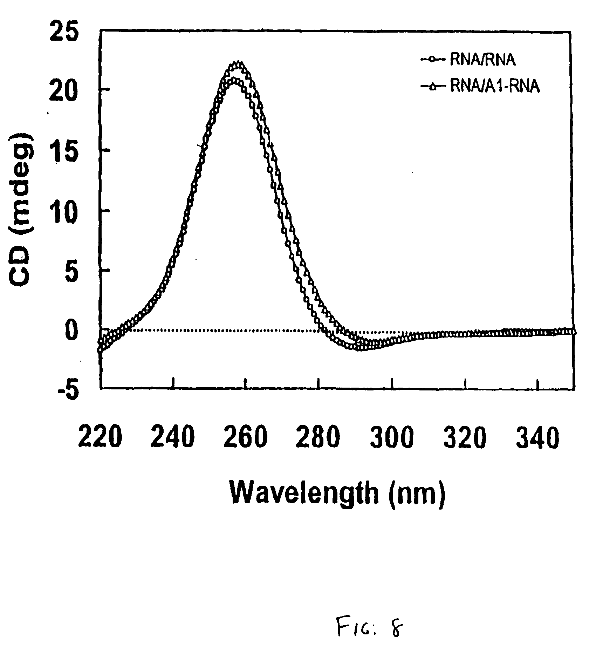

CD spectral analysis were used to determine whether incorporation of cyclohexenylA has a fundamental influence on the conformation of a double helical structure. A comparison was made between the CD spectra of the duplexes containing three A1 residues, the natural reference duplexes and the same complexes having three A₂ residues incorporated. It was demonstrated that oligo (A₁) and oligo(dT) form a complex in a 1:2 ratio. The CD spectra ofthe dsDNA sequences (Figure 6) are characterized by positive cotton effect at 264 nm and a negative cotton effect at 241 nm. After incorporation of three A₁ residues (A₁-DNA/DNA), the spectrum is similar but not the same. The positive CD band at 264 nm is more intense while the negative CD band at 238 nm is less intense. This trend is continuing when three A₂ residues are incorporated (A₂-DNA/DNA). The negative signal at 238 nm becomes weaker while the intensity of the positive band at 264 nm increases. The spectrum of the A₂-DNA/DNA complex becomes more similar to the DNA/RNA duplex while the spectrum of the A₁-DNA/RNA complex is intermediate between that of a DNA/RNA and RNA/DNA duplex. This is a first indication that the structures of A₁-DNA/DNA and of A₂₋DNA/DNA are different. The CD spectra of the complexes: RNA/A₁=DNA; RNA/A₂-DNA; DNA/RNA; RNA/DNA and DNA/A₁-RNA are very similar (Figure 7). An intense positive CD band is observed around 260 nm while nearly no negative signals (or bands of very low intensity) are present. The intensity of the positive band decreases in the order given. All complexes seem to have the same general geometry. The same can be said from the spectra of the dsRNA and RNA/A₁-RNA complexes (Figure 8), although the positive band is somewhat shifted to shorter wavelength. The CD spectral analysis demonstrate similar conformation for all RNA containing complexes. Three A₂ residues incorporated in a DNA chain seem to induce a conformational change of a dsDNA duplex in the direction of a DNA/RNA type structure. Three A₁ residues do not seem to have this effect and the DNA/A₁₋DNA conformation stays close to the typical dsDNA form. This might be explained by a different conformation of cyclohexenylA in a dsDNA and in a DNA/RNA duplex. Conformational changes (chair inversion) of A₂ costs a lot of energy, while A1 is expected to undergo more easily half-chair inversion.

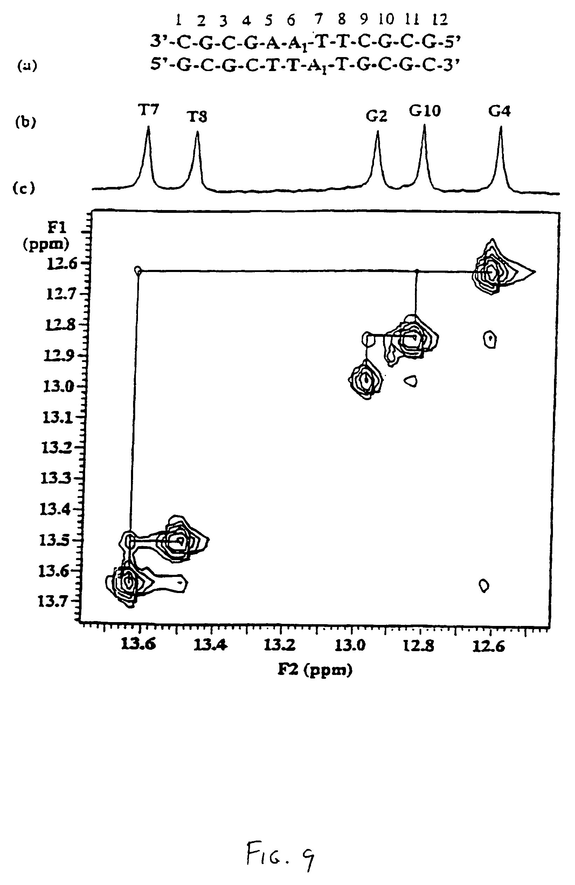

In order to study the effect of incorporation of cyclohexenylA on the conformation of a DNA duplex in more details we incorporated one D-cyclohexenylA (A₁) residue in the well-known self-complementary Dickerson dodecamer, 5'-d [CGCGAA₁TTCGCG]₂-3' (I), and investigated the solution state conformation using NMR. Under the appropriate experimental conditions (1.4 mM oligomer, 10mM KH₂PO₄, 10 µM EDTA, pH 7.5 in 90% H₂O/10%D₂O), the imino protons of guanine and thymine give rise to NMR peaks in the range of 12-14 ppm in a ID WATERGATE spectrum (Fig. 8b). In a 2D WATERGATE NOESY 17 spectrum (Fig. 8c) the diagonal peaks match the imino protons of the ID spectrum (Fig 8b). Each Watson-Crick G-C and A-T base-pair has one imino proton involved in hydrogen bonding. The terminal G-imino resonance is not seen because of fraying. The imino protons ofneighboring base-pairs in a base-paired helical region are close enough to give NOE crosspeaks in a 2D spectrum and in Fig. 8c, the imino-imino NOE crosspeaks between the neighboring base-pairs are very clear. The NOE connectivity walks on the 2D spectrum (Fig. 8c) and the sequential imino proton assignments correspond with a symmetric duplex 9Fig. 8a). NOEs for the adenine H2 (AH2) of the A-T base-pairs to their own T imino proton and to the imino protons of neighboring pairs prove additionally the presence of a duplex. Non-exchangeable proton assignments are based on an analysis of through space NOESY, through bond COSY, TOCSY and ¹H-³¹P HETCOR datas using well described procedures for DNA duplexes. Fig. 9b shows a fragment of a NOESY spectrum (250 ms) of the duplex (I) at 20 °C in D₂O showing crosspeaks between the base protons (H6 in pyrimidines or H8/H2 in purines) and anomeric protons and pyrimidine H5 protons which was used to assign base (H8/H6/H2) and H1' protons in a sequential manner. The sequential walk was disrupted by the absence of the A6H1'-T7H6 crosspeak. Figure 18 contains the aromatic and H1' proton chemical shifts of the modified duplex (I) at 20 °C and 25 °C and of the unmodified duplex (II) 5'-d [CGCGAATTCGCG]2-3. Chemical shift changes are indicative of structural changes associated with the introduction of modified nucleotides. Figure 18, however, shows negligible changes in chemical shift (max 0.1 ppm difference) for all the other residues implying that A1 does not disturb the B-structure of the duplex. The changes are limited to the modified nucleotide itself. Figure 11 shows a NOESY-COSY connectivity diagram for sequential assignments via a NOSEY spectrum(50ms) between base protons and 2'C protons using NOESY with a mixing time of 50 ms and a DQF COSY spectrum which contains the crosspeaks between 1'H and 2'-CH2. The observation that (i), most of the H2" protons are more downfield in chemical shift than the H2' protons; (ii),the intraresidue and interresidue NOES between H8/H6 and H2' and H2" respectively are more intense than the intraresidue NOES between H8/H6 and H2" and (iii), the H1'-H2', H1-H2" crosspeaks have characteristic B-like phase-sensitive multiplets, confirms as well the existence of a regular B-type DNA structure.

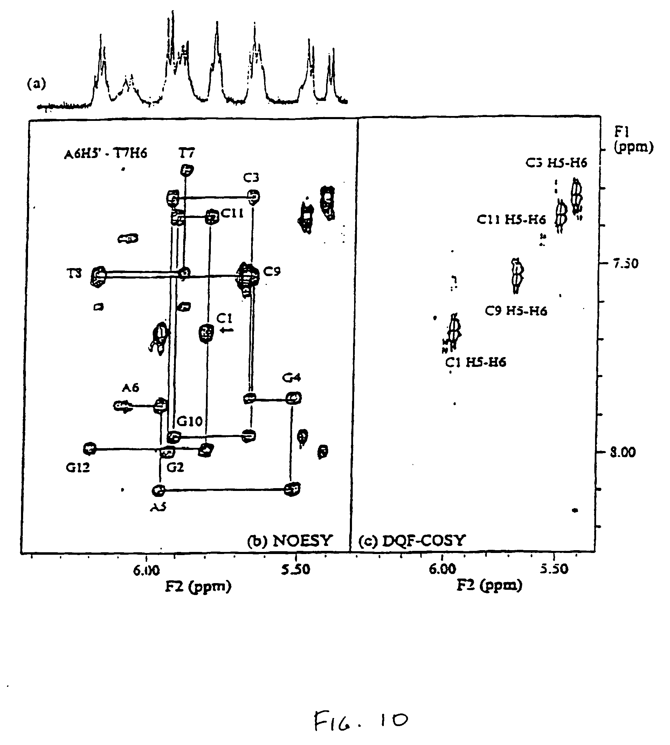

However the NOESY spectrum (Fig. 9b) and the DQF COSY spectrum (Fig. 9c), representing the region of crosspeaks between the base protons (H6/H8) and anomeric protons (H1') and between H5 and H6 protons of the pyrimidine residues and the H1'/H5 proton region of the ID spectrum (Fig. 9a) show the presence of broad proton crosspeaks and multiple resonances (some of them are highlighted with a grey box in Fig. 9b), suggesting the presence of multiple species. No peak sharpening is observed by varying the temperature over the range of 25 to 40 C, or by changing the salt concentration. The existence of more than one structure makes it difficult to determine a highly refined structure by using NMR methods. Another remarkable feature ofthis duplex (I) is the total absence of H1'-H2'/H2"; H3-H2'/H2" and H3'-H4' crosspeaks (Fig. 10b) and the presence of only very weak H4'-H6', H4'-H5' crosspeaks (marked with an asterisk in Fig. 10b) for the CyclohexenylA residue in the DQF COSY and the TOCSY spectra. The NOESY A6H8-H2'/H2" crosspeaks are also very weak. The peaks are probably too broad to be detected. In conclusion, the incorporation of the CyclohexenylA residue (A1) has practically no effect on the global structure of the Dickerson dodecamer and changes manifest themselves only in the modified 6^{th} residue (A1). However, the line broadening and multiple resonances suggest that the cyclohexenylA (A1) residue induces confonnational mobility which precludes a detailed structural analysis.

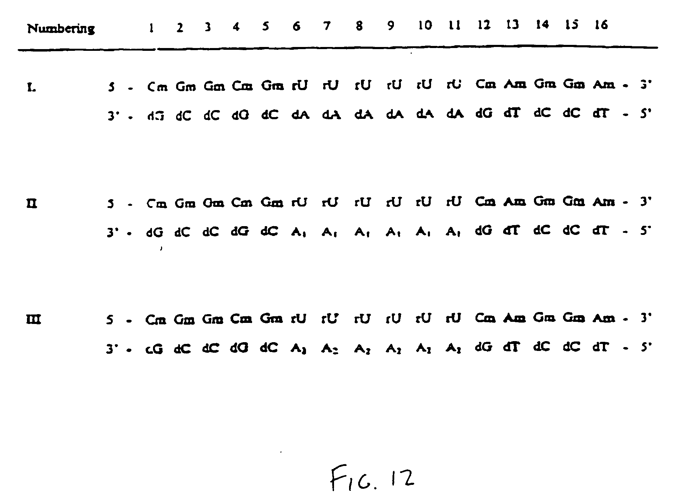

We used a double chimeric approach to evaluate the RNase H susceptibility of the CeNA/RNA duplex (Figure 12). We synthesized a chimeric 2'-O-methyl RNA-RNA oligonucleotide that is complementary to the chimeric DNA-CeNA oligonucleotide (Figure 12, II). We can not use a full RNA oligonucleotide as complement of the DNA-CeNA chimera because strand cleavage may then occur outside the CeNA/RNA region. It has been reported that 2'-O-methyl RNA/DNA hybrids are not substrates for RNase H and that partially modified chimeric oligonucleotides containing deoxy gaps of 5 residues or longer activate RNase H in vitro, although less efficiently than the parent uniform deoxynucleotide. In addition, the sequence-specific variations in the structure ofDNA/RNA hybrids affect groove widths and cleavage susceptibility by RNase H. It has been observed for example that local segments of polypurine-RNA/DNA duplexes are resistant to cleavage by the RNase H activity of reverse transcriptase. We also observed experimentally that oligoadenylate-poly(rU) complexes [(dA) 13:poly(rU); are poor substrate for RNase H of E. Coli.

A chimeric oligo (rU)/oligo(dA) duplex was used as a positive control (Figure 12, 1) to study the cleavage of the rU strand by RNase H, and the double chimeric duplex as described for the CeNA oligomer (HNA replacing CeNA) was used as a negative control (Figure 12, III). E. Coli enzyme was used for the study of the susceptibility of the hybrids to be cleaved by RNase H because this enzyme is readily available and its cleavage property is not so different from the mammalian enzyme.

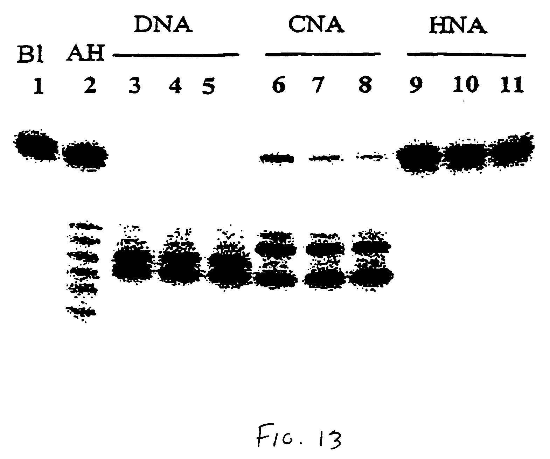

Figure 13 shows the cleavage pattern ofthe three complexes (Figure 12, I-III) after incubation for 1 h. 3h and 6h with E. Coli RNase H. The complex I is quickly degraded, while the HNA containing complex formed between the cyclohexenyl oligomer and its RNA complement is an efficient substrate for RNase H. Two cleavages sites in the RNA strand are observed in complex II, i.e. one between (U)₇ and (U)₈ and the other between (U)₉ and (U)₁₀. It is remarkable that the cleavage pattern in an oligo(rU) sequence is dependent on the sugar modification in the complementary A-tract. Indeed, the complex I (Figure 12) is not only somewhat faster degraded, but cleavage occurs preferentially between (U)₈ and (U)₉ and between (U)₉ and (U)₁₀. Displacement of A₂ by the conformational more mobile A₁ results in a large difference in cleavage susceptibility.

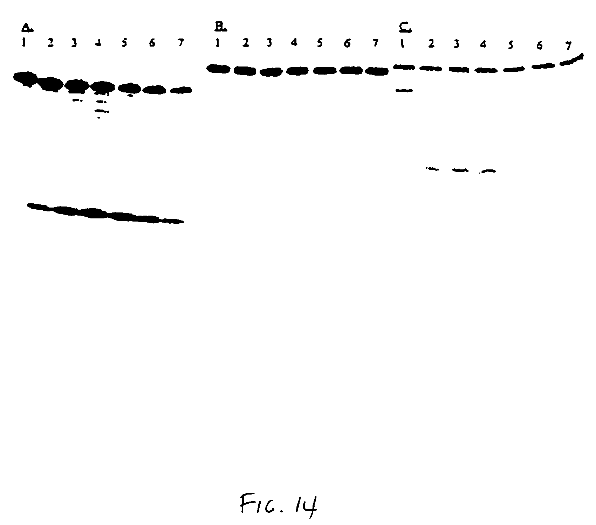

The stability of the modified oligonucleotide was likewise tested in serum (Figure 14). This experiment shows that CeNA has serum stability similar to HNA. Both modified oligonucleotides are much more stable against enzymatic degradation (no degradation after incubation for 3h in serum 37°C) than DNA (almost half degraded after 3h incubation). CeNa combines the interesting future of serum stability and the properties to induce RNase H.

An ideal antisense oligonucleotide is one that hybridizes strongly with its RNA complement and able to induce RNase H-cleavage of his target. Strong hybridization of charged nucleic acids to RNA means preorganization in an A-type duplex for maximal interstrand stacking. Duplex stabilization has been observed with several carbohydrate modified oligonucleotides. From this research it can be concluded that the more the nucleoside building block is preorganized in an N-type furanose conformer, the higher the stability is of the duplex formed between the modified oligomer and its RNA complement. However, the minor groove width of stable A-type duplexes is too large for efficient recognition by RNase H. It seems therefore that a compromise between both might be the best solution and that a rather modest increase in duplex stabilization (based on a preorganization in for example an O4'-endo conformation) might be compatible with RNase H activation. RNase H is activated by RNA/DNA hybrids in which the DNA strand represents phosphodiester- or phosphorothioate oligonucleotides. The latter oligomers have a modified "phosphate" internucleoside linkage. As far as we know, the only sugar-modified oligonucleotides that activate (E. Coli) RNase H are arabinonucleic acids with either a 2'-hydroxyl- or a 2'-fluoro substituent. Oligonucleotide-arabinonucleic acids (2'-OH) destabilize the RNA/DNA duplex considerably and have no advantage over phosphorothioate oligonucleotides.

The cyclohexene nucleic acids of the invention, when incorporated into a DNA sequence, increase the stability of the duplex formed between this DNA and its complementary RNA. For the studied sequence, the ΔTm/mod averages+1.2 °C. The capacity of a cyclohexene nucleotide to undergo conformational changes when incorporated in an oligonucleotide was evaluated at three different levels. The complex between CeNA (oligonucleotide A) and DNA (oligo dT) is of triple helical origin and has a stability similar to the complex between CeNA (oligonucleotide A) and RNA (oligonucleotide rU) (which is not the case for HNA/DNA and HNA/RNA). Secondly, the CD spectrum of a A₁-DNA/DNA duplex is similar to the spectrum ofdsDNA (which is not the case for A₂-DNA/DNA duplex). The CD spectrum of a RNA/A₁-DNA is similar to the spectrum of RNA/DNA and the CD spectrum of RNA/A₁-RNA and of dsRNA are identical. These results indicate that a cyclohexene nucleotide can easily be accommodated in different nucleic acid structures. The structural relationship between A₁ and a natural furanose nucleoside might be fundamental for these properties. Thirdly, NMR analysis of a Dickerson dodecamer containing A₁ suggest conformational mobility of this residue.

The cyclohexene nucleic acid when hybridized to RNA is, likewise, able to activate RNase H. In contrast, the HNA/RNA duplex is not degraded. It might be expected that the minor groove width of the CeNA/RNA duplex is comparable to that of a DNA/RNA duplex (9A) and that RNase H can efficiently bind to the complex dsRNA with a minor groove width of 11A is not degraded at all. CeNA/RNA duplexes are thermally more stable than the natural counterparts and that CeNA is stable against degradation in serum. Therefore, CeNA can be considered as a new oligonucleotide combining the advantage of duplex stabilization with the potential to activate RNase H, which means that these oligomers are prime candidates for evaluation as antisense constructs in a cellular system.

As is known in the art, a nucleoside typically includes a covalently bound sugar and base. The base portion of the nucleoside is normally a heterocyclic base. The two most common classes of such heterocyclic bases are the purines and the pyrimidines. For nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. The respective ends of this linear polymeric structure can be open linear structures or can be joined to form a circular structure.

Within the oligonucleotide structure, the phosphate groups are commonly referred to as forming the intersugar "backbone" of the oligonucleotide. The normal linkage or backbone of RNA and DNA is a 3' to 5' phosphodiester linkage. The backbone of an oligonucleotide (or other antisense compound) positions a series of bases in a specific order; the written representation of this ordered series of bases, usually written in 5' to 3' order unless otherwise indicated, is known as a nucleotide or nucleobase sequence.

Oligonucleotides may comprise nucleotide sequences sufficient in identity and number to effect specific hybridization with a particular nucleic acid. Such oligonucleotides which specifically hybridize to a portion of the sense strand of a gene are commonly described as "antisense." In the context of the invention, "hybridization" means hydrogen bonding, which may be Watson-Crick, Hoogsteen or reversed Hoogsteen hydrogen bonding, between complementary nucleotides. For example, adenine and thymine are complementary nucleobases which pair through the formation of hydrogen bonds. "Complementary," as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other.

"Specifically hybridizes" and "complementary" are thus terms which are used to indicate a sufficient degree of complementarity or precise pairing such that stable and specific binding occurs between the oligonucleotide and the DNA or RNA target. An oligonucleotide specifically hybridizes to its target sequence due to the formation ofbase pairs between specific partner nucleobases in the interior of a nucleic acid duplex. Among the naturally occurring nucleobases, guanine (G) binds to cytosine (C), and adenine (A) binds to thymine (T) or uracil (U). In addition to the equivalency of U (RNA) and T (DNA) as partners for A, other naturally occurring nucleobase equivalents are known, including 5-methylcytosine and 5-hydroxymethylcytosine (HMC) (c) equivalents), and 5-hydroxymethyluracil (U equivalent). Furthermore, synthetic nucleobases which retain partner specificity are known in the art and include, for example, 7-deaza-Guanine, which retains partner specificity for C. Thus, an oligonucleotide's capacity to specifically hybridize with its target sequence will not be altered by a chemical modification to a nucleobase in the nucleotide sequence of the oligonucleotide which does not impact its specificity for a partner nucleobase in the target nucleic acid.

It is understood in the art that the nucleobase sequence of an oligonucleotide or other antisense compound need not be 100% complementary to its target nucleic acid sequence to specifically hybridize. An antisense compound specifically hybridizes to its target nucleic acid when there is a sufficient degree of complementarity to avoid non-specific binding of the oligonucleotide to non-target sequences under conditions in which specific binding is desired, *i.e.,* under physiological conditions in the case of *in vivo* assays or therapeutic treatment, or, in the case of *in vitro* assays, under assay conditions.

Antisense oligonucleotides are commonly used as research reagents, diagnostic aids, and therapeutic agents. For example, antisense oligonucleotides, which are able to inhibit gene expression with exquisite specificity, are often used by those of ordinary skill to elucidate the function of particular genes, for example to distinguish between the functions of various members of a biological pathway. This specific inhibitory effect has, therefore, been harnessed by those skilled in the art for research uses. The specificity and sensitivity of oligonucleotides is also harnessed by those of skill in the art for therapeutic uses.

The present invention provides for therapeutic and pharmaceutical compositions comprising cyclohexene nucleic acids. Compositions for the administration of the cyclohexene nucleic acids of the invention may include sterile aqueous solutions which may also contain buffers, diluents and other suitable additives.

The pharmaceutical compositions of the invention, which may conveniently be presented in unit dosage form, may be prepared according to conventional techniques well known in the pharmaceutical industry. Such techniques include the step of bringing into association the active ingredient(s) with the pharmaceutically acceptable carriers). In general the pharmaceutical compositions are prepared by uniformly and intimately bringing into association the active ingredient(s) with liquid excipients or finely divided solid excipients or both, and then, if necessary, shaping the product.

Pharmaceutical and therapeutic compositions comprising one or more of the antisense compounds of the invention may further include sterile aqueous solutions which may also contain buffers, diluents and other suitable additives. Pharmaceutically acceptable organic or inorganic carrier substances suitable for non-parenteral administration which do not deleteriously react with the antisense compounds can be used. The pharmaceutical compositions can be sterilized and, if desired, mixed with auxiliary agents, e.g., lubricants, preservatives, stabilizers, wetting agents, emulsifiers, salts for influencing osmotic pressure, buffers, colorings flavorings and/or aromatic substances and the like which do not deleteriously react with the oligonucleotide(s) of the pharmaceutical composition. Pharmaceutical compositions in the form of aqueous suspensions may contain substances which increase the viscosity of the suspension including, for example, sodium carboxymethylcellulose, sorbitol and/or dextran. Optionally, such compositions may also contain one or more stabilizers, carrier compounds or pharmaceutically acceptable carriers.

As used herein, "carrier compound" refers to a compound, which is inert (i.e., does not possess biological activity per se) but is recognized by *in vivo* processes that reduce the bioavailability of a bioactive agent by, for example, degrading the bioactive agent or promoting its removal from circulation. The coadministration of a bioactive agent and a carrier compound, typically with an excess of the latter substance, can result in a substantial reduction of the amount of bioactive agent recovered in the liver, kidney or other extracirculatory reservoirs, presumably due to competition between the carrier compound and the bioactive agent for a common receptor. For example, the recovery of a partially phosphorothioated oligonucleotide in hepatic tissue is reduced when it is coadministered with polyinosinic acid, dextran sulfate, polycytidic acid or 4-acetamido-4'-isothiocyano-stilbene-2,2'-disulfonic acid (Miyao et al., Antisense Res. Dev., 1995, 5, 115; Takakura et al., Antisense & Nucl. Acid Drug Dev., 1996, 6, 177).

In contrast to a carrier compound, a "pharmaceutically acceptable carrier" (excipient) is a pharmaceutically acceptable solvent, suspending agent or any other pharmacologically inert vehicle for delivering one or more bioactive agents to an animal. The pharmaceutically acceptable carrier may be liquid or solid and is selected with the planned manner of administration in mind so as to provide for the desired bulk, consistency, etc., when combined with a nucleic acid and the other components of a given pharmaceutical composition. Typical pharmaceutically acceptable carriers include, but are not limited to, binding agents (e.g., pregelatinised maize starch, polyvinylpyrrolidone or hydroxypropyl methylcellulose, etc.); fillers (e.g., lactose and other sugars, microcrystalline cellulose, pectin, gelatin, calcium sulfate, ethyl cellulose, polyacrylates or calcium hydrogen phosphate, etc.); lubricants (e.g., magnesium stearate, talc, silica, colloidal silicon dioxide, stearic acid, metallic stearates, hydrogenated vegetable oils, corn starch, polyethylene glycols, sodium benzoate, sodium acetate, etc.); disintegrates (e.g., starch, sodium starch glycolate, etc.); or wetting agents (e.g., sodium lauryl sulphate, etc.). Suitable pharmaceutically acceptable carriers include, but are not limited to, water, salt solutions, alcohol, polyethylene glycols, gelatin, lactose, amylose, magnesium stearate, talc, silicic acid, hydroxymethylcellulose, polyvinylpyrrolidone viscous paraffin and the like.

The compositions of the present invention may additionally contain other adjunct components conventionally found in pharmaceutical compositions, at their art-established usage levels. Thus, for example, the compositions may contain additional compatible pharmaceutically-active materials such as, e.g., antipruritics, astringents, local anesthetics or anti-inflammatory agents, or may contain additional materials useful in physically formulating various dosage forms of the composition of present invention, such as dyes, flavoring agents, preservatives, antioxidants, opacifiers, thickening agents and stabilizers. However, such materials, when added, should not unduly interfere with the biological activities of the components of the compositions of the invention.

The administration of therapeutic or pharmaceutical compositions comprising the cyclohexene nucleic acid of the invention is believed to be within the skill of those in the art. In general, a patient in need of therapy or prophylaxis is administered a composition comprising a cyclohexene nucleic acid in accordance with the invention, commonly in a pharmaceutically acceptable carrier, in doses ranging from 0.01 ug to 100 g per kg of body weight depending on the age of the patient and the severity of the disorder or disease state being treated. Dosing is dependent on severity and responsiveness of the disease state to be treated, with the course of treatment lasting from several days to several months, or until a cure is effected or a diminution or prevention of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual antisense compounds, and can generally be estimated based on EC50s found to be effective in vitro and in vivo animal models.

In the context of the invention, the term "treatment regimen" is meant to encompass therapeutic, palliative and prophylactic modalities of administration of one or more pharmaceutical compositions of the invention. A particular treatment regimen may last for a period of time which will vary depending upon the nature of the particular disease or disorder, its severity and the overall condition of the patient, and may extend from once daily to once every 20 years. Following treatment, the patient is monitored for changes in his/her condition and for alleviation of the symptoms of the disorder or disease state. The dosage of the pharmaceutical composition may either be increased in the event the patient does not respond significantly to current dosage levels, or the dose may be decreased if an alleviation of the symptoms of the disorder or disease state is observed, or if the disorder or disease state has been ablated.

An optimal dosing schedule is used to deliver a therapeutically effective amount ofthe pharmaceutical composition ofthe invention being administered via a particular mode of administration. The term "therapeutically effective amount," for the purposes of the invention, refers to the amount of oligonucleotide-containing pharmaceutical composition which is effective to achieve an intended purpose without undesirable side effects (such as toxicity, irritation or allergic response). Although individual needs may vary, determination of optimal ranges for effective amounts of pharmaceutical compositions is within the skill of the art. Human doses can be extrapolated from animal studies (Katocs et al., Chapter 27 In: Remington's Pharmaceutical Sciences, 18th Ed., Gennaro, ed., Mack Publishing Co., Easton, PA, 1990). Generally, the dosage required to provide an effective amount of a pharmaceutical composition, which can be adjusted by one skilled in the art, will vary depending on the age, health, physical condition, weight, type and extent of the disease or disorder of the recipient, frequency of treatment, the nature of concurrent therapy (if any) and the nature and scope of the desired effect(s) (Nics et al., Chapter 3 In: Goodman & Gilman's The Pharmacological Basis of Therapeutics, 9th Ed., Hardman et al., eds., McGraw-Hill, New York, NY, 1996).

Following successful treatment, it may be desirable to have the patient undergo maintenance therapy to prevent the recurrence ofthe disease state, wherein the pharmaceutical composition is administered in maintenance doses, ranging from 0.01 ug to 100 g per kg of body weight, once or more daily, to once every 20 years. For example, in the case of in individual known or suspected of being prone to an autoimmune or inflammatory condition, prophylactic effects may be achieved by administration of preventative doses, ranging from 0.01 ug to 100 g per kg of body weight, once or more daily, to once every 20 years. In like fashion, an individual may be made less susceptible to an inflammatory condition that is expected to occur as a result of some medical treatment, e.g., graft versus host disease resulting from the transplantation of cells, tissue or an organ into the individual.

Prophylactic modalities for high risk individuals are also encompassed by the invention. As used herein, the term "high risk individual" is meant to refer to an individual for whom it has been determined, via, e.g., individual or family history or genetic testing, that there is a significantly higher than normal probability of being susceptible to the onset or recurrence of a disease or disorder. As part of a treatment regimen for a high risk individual, the individual can be prophylactically treated to prevent the onset or recurrence of the disease or disorder. The term "prophylactically effective amount" is meant to refer to an amount of a pharmaceutical composition which produces an effect observed as the prevention of the onset or recurrence of a disease or disorder. Prophylactically effective amounts of a pharmaceutical composition are typically determined by the effect they have compared to the effect observed when a second pharmaceutical composition lacking the active agent is administered to a similarly situated individual.

The therapeutic and pharmaceutical compositions of the present invention may be administered in a number of ways depending upon whether local or systemic treatment is desired and upon the area to be treated. Typically, parenteral administration is employed. The term "parenteral delivery" refers to the administration of one or more antisense compounds of the invention to an animal in a manner other than through the digestive canal. Parenteral administration includes intravenous (i.v.) drip, subcutaneous, intraperitoneal (i.p.) or intramuscular injection, or intrathecal or intraventricular administration. Compositions for parenteral, intrathecal or intraventricular administration may include sterile aqueous solutions which may also contain buffers, diluents and other suitable additives. Means of preparing and administering parenteral pharmaceutical compositions are known in the art (see, e.g., Avis, Chapter 84 In: Remington's Pharmaceutical Sciences, 18th Ed., Gennaro, ed., Mack Publishing Co., Easton, PA, 1990, pages 1545-1569). Parenteral means of delivery include, but are not limited to, the following illustrative examples.

Intravenous administration of pharmaceutical formulations comprising bioactive agents is well known in the art. Bioactive agents delivered i.v. include an anticancer drug, doxorubicin, and an antifungal drug, amphotericin B (see, e.g., Riaz et al., Chapter 16 in Pharmaceutical Dosage Forms: disperse Systems, Vol. 2, Lieberman et al., eds., Marcel Dekker, Inc., New York, NY, 1989, pages 567-603). With particular regards to antisense compounds, i.v. administration of such compounds to various non-human mammals has been described by Iversen (Chapter 26 In: Antisense Research and Applications, Crooke et al., eds., CRC Press, Boca Raton, FL, 1993, pages 461-469), and systemic delivery ofoligonucleotides to non-human mammals via intraperitoneal means has also been described (Dean et al., Proc. Natl. Acad. Sci. USA, 1994, 91, 11766).

Intraluminal drug administration, for the direct delivery of drug to an isolated portion of a tubular organ or tissue (e.g., such as an artery, vein, ureter or urethra), may be desired for the treatment of patients with diseases or conditions afflicting the lumen of such organs or tissues. To effect this mode of administration, a catheter or cannula is surgically introduced by appropriate means. For example, for treatment of the left common carotid artery, a cannula is inserted thereinto via the external carotid artery. After isolation of a portion of the tubular organ or tissue for which treatment is sought, a composition comprising the antisense compounds of the invention is infused through the cannula or catheter into the isolated segment. After incubation for from about 1 to about 120 minutes, during which the bioactive agent is taken up by cells of the interior lumen of the vessel, the infusion cannula or catheter is removed and flow within the tubular organ or tissue is restored by removal of the ligatures which effected the isolation of a segment thereof (Morishita et al., Proc. Natl. Acad. Sci. U.S.A., 1993, 90, 8474). Bioactive agents may also be combined with a biocompatible matrix, such as a hydrogel material, and applied directly to vascular tissue in vivo (Rosenberg et al., U.S. Patent No. 5,593,974, issued January 14, 1997).

Intraventricular drug administration, for the direct delivery of drug to the brain of a patient, may be desired for the treatment of patients with diseases or conditions afflicting the brain. To effect this mode of administration, a silicon catheter is surgically introduced into a ventricle of the brain of a human patient, and is connected to a subcutaneous infusion pump (Medtronic Inc., Minneapolis, MN) that has been surgically implanted in the abdominal region (Zimm et al., Cancer Research, 1984, 44, 1698; Shaw, Cancer, 1993, 72(11 Suppl., 3416). The pump is used to inject the bioactive agent and allows precise dosage adjustments and variation in dosage schedules with the aid of an external programming device. The reservoir capacity of the pump is 18-20 mL and infusion rates may range from 0.1 mL/h to 1 mL/h. Depending on the frequency of administration, ranging from daily to monthly, and the dose of drug to be administered, ranging from 0.01 ug to 100 g per kg of body weight, the pump reservoir may be refilled at 3-10 week intervals. Refilling of the pump is accomplished by percutaneous puncture of the pump's self-sealing septum.

Intrathecal drug administration, for the introduction of a drug into the spinal column of a patient may be desired for the treatment of patients with diseases of the central nervous system (CNS). To effect this route of administration, a silicon catheter is surgically implanted into the L3-4 lumbar spinal interspace of a human patient, and is connected to a subcutaneous infusion pump which has been surgically implanted in the upper abdominal region (Luer and Hatton, The Annals of Pharmacotherapy, 1993, 27, 912, 1993; Ettinger et al. Cancer, 1978, 41, 1270; Yaida et al., Regul. Pept., 1985, 59, 193). The pump is used to inject the pharmaceutical composition and allows precise dosage adjustments and variations in dose schedules with the aid of an external programming device. The reservoir capacity of the pump is 18-20 mL, and infusion rates may vary from 0.1 mL/h to 1 mL/h. Depending on the frequency of drug administration, ranging from daily to monthly, and dosage of drug to be administered, ranging from 0.01 ug to 100 g per kg of body weight, the pump reservoir may be refilled at 3-10 week intervals. Refilling of the pump is accomplished by a single percutaneous puncture to the self-sealing septum of the pump. The distribution, stability and pharmacokinetics of a bioactive agent (such as an oligonucleotide) within the CNS are followed according to known methods (Whitesell et al., Proc. Natl. Acad. Sci. USA,1993, 90, 4665).

To effect delivery of bioactive agents to areas other than the brain or spinal column via this method, the silicon catheter is configured to connect the subcutaneous infusion pump to, e.g., the hepatic artery, for delivery to the liver (Kemeny et al., Cancer, 1993, 71, 1964). Infusion pumps may also be used to effect systemic delivery of bioactive agents (Ewel et al., Cancer Res., 1992, 52, 3005; Rubenstein et al., J. Surg. Oncol., 1996, 62, 194).

Vaginal delivery provides local treatment and avoids first pass metabolism, degradation by digestive enzymes, and potential systemic side-effects. This mode of administration may be preferred for bioactive agents targeted to pathogenic organisms for which the vagina is the usual habitat, e.g., Trichomonas vaginalis. Vaginal suppositories (Block, Chapter 87 In: Remington's Pharmaceutical Sciences, 18th Ed., Gennaro, ed., Mack Publishing Co., Easton, PA, 1990, pages 1609-1614) or topical ointments can be used to effect this mode of delivery.

Intravesical delivery provides local treatment and avoids first pass metabolism, degradation by digestive enzymes, and potential systemic side-effects. However, the method requires urethral catheterization of the patient and a skilled staff. Nevertheless, this mode of administration may be preferred for bioactive agents targeted to pathogenic organisms, such as T. vaginalis, which may invade the urogenital tract.

Intravitreal injection, for the direct delivery of drug to the vitreous humor of a mammalian eye, is described in U.S. Patent No. 5,591,720, the contents of which are hereby incorporated by reference. Means ofpreparing and administering ophthalmic preparations are known in the art (see, e.g., Mullins et al., Chapter 86 In: Remington's Pharmaceutical Sciences, 18th Ed., Gennaro, ed., Mack Publishing Co., Easton, PA,1990, pages 1581-1595).

### Experimental

RNaseUltraviolet spectra were recorded with Philips PU8740 UV/Vis spectrophotometer. The ¹H NMR and ¹³C NMR spectra were recorded with a Varian Gemini 200 spectrometer. Tetramethylsilane was used as internal standard for the ¹H NMR spectra (s= singlet, d=doublet, t=triplet, m=multiplet) CDCl₃ (δ = 76.9) or CD₃OD (δ =49.0) for the ¹³C NMR spectra. Mass spectrometry measurements were obtained using a Kratos Concept 1H mass spectrometer. Pyridine was refluxed overnight on potassium hydroxide and distilled. Dichloromethane was stored on calcium hydride, refluxed and distilled. Precoated Macherey-Nagel Alugram® SIL G/UV 254 plates were used for TLC and the products were visualized with UV light. Column chromatography was performed on Acros Chimica silica gel (0.2-0.5 mm, pore size 4 nm).

### N6-Benzoyl-9-[(1S,4R,SS)-5-hydroxyl-4-hydroxymethyl-2-cyclohexeneyl]adenine (2)

To a solution of 1 (500 mg, 1.67 mmol, dried three times with dry pyridine) in dry pyridine (20mL) at 0 °C under nitrogen was added dropwise TMSCI (1.06 mL, 8.38 mmol, 5 eq.). After stirring for 1 hour, BzCl (0.97 mL, 8.38 mmol, 5 eq.) was added slowly. The reaction mixture was stirred for 1 hour at 0 °C, warmed up to room temperature and stirred for additional 13 hours. The reaction mixture was then cooled in an ice-bath and quenched with water (3 mL). After stirring for 5 minutes, 25% NH₄OH solution (5 mL) was added. The resulting mixture was stirred at room temperature for 15 minutes and concentrated. The residue was dissolved in MeOH (15 mL) and 25% NH₄OH solution (10mL) and stirred at room temperature for 0.5 hours. After concentration, the residue was purified on silica gel (CH₂Cl₂-MeOH 50:1, 20:1, and 10: 1) to afford 2 (385 mg, 63%) as white foams. ¹H NMR (DMSO-*d*_{*6*}) δ 2.01 (m, 1H), 2.15 (m, 2H), 3.60 (m, 2H), 3.78 (m, 1H), 4.76 (t, 1H, *J* =5.1 Hz, -CH₂OH), 5.42 (m, 1H), 5.88 (dm,1H, *J* =9.9 Hz), 6.05 (dm, 1 H, *J =* 9.9 Hz), 7.59 (m, 3H), 8.04 (m, 2H), 8.34 (s, 1H), 8.76 (s, 1H), 11.17 (br-s, 1H, -NH); ¹³C NMR (CD₃OD) δ 36.9 (t), 47.7 (d), 63.1 (t), 64.9 (d), 125.3 (d) 129.5 (d), 129.9 (d), 134.0 (d), 135.1 (s), 136.1 (d) 144.8 (d), 151.2 (s), 153.1 (d), 153.3 (s), 168.3 (s); UV λₘₐₓ (MeOH) = 280 nm; LISMS (THGLY) 366 (M+H)⁺;HRMS calcd for C₁₉H₂₀N₅O₃ (M+H)⁺ 366.1566, found 366.1571.

### N6-Benzoyl-9-[(1S,4R,SS)-5-hydroxy-4-monomethoxytrityloxymethyl-2-cyclohexeneyl]-adenine (3)

To a solution of 2 (240 mg, 0.66 mmol, dried three times with dry pyridine) in dry pyridine (5 mL) at 0 °C under nitrogen was added MMTrCl (305 mg, 0.99 mmol, 1.5 eq.) in portions. After stirring at 0 °C for 0.5 hours, the reaction was warmed up to room temperature and kept at this temperature for 22 hours. The reaction mixture was cooled in an ice bath and treated with MeOH (5 mL). After stirring at room temperature for 0.5 hours, the resulting mixture was concentrated. The residue was coevaporated with toluene to remove traces of pyridine and chromatographed on silica gel. (CHCl₃-MeOH 100:1, 1%Et₃N) to afford 3 (240 mg, 57%) as white foam. 1H NMR (CDC13) δ2.12 (m, 2H), 2.54 (m, 1H), 3.24 (t, 1Hm*J*= 8.8 Hz), 3.49 (s, 1H, -OH), 3.52 (dd, 1H, *J* = 8.8, 4.4 Hz), 3.78 (s, 3H), 3.85 (m, 1H), 5.43 (m, 1H), 5.82 - 5.96 (m, 2H), 6.84 (d, 2H, *J=* 7.8 Hz), 7.19 - 7.61 (m, 15), 7.88 (s, 1H), 8.01(d, 1H, *J =* 7.3 Hz), 8.75 (s, 1H), 9.35(s, 1H, - NH); ¹³C NMR (CDCl₃) δ 35.6 (t), 44.3 (q), 49.5 (d), 55.2 (d), 65.6 (t), 66.4 (d), 87.2 (s), 113.3 (d), 123.4 (s), 123.9 (d), 127.2 (d), 128.0 (d), 128.3 (d), 128.8 (d), 130.2 (d), 132.7 (d), 133.8 (s), 133.9 (d) 135.1 (s), 141.8 (d), 143.9 (s), 144.0 (s), 149.6 (s), 151.6 (s),152.4 (d), 158.8 (s), 164.8 (s); UV λₘₐₓ (MeOH) = 280, 254nm; LISMS (THGLU/NBA) 660 (M+Na)⁺; HRMS calcd for C₃₉H₃₅N₅O₄Na (M+Na)⁺ 660.2587, found 660.2589.

### Preparation of the amidite monomer 4

The modified nucleoside 3 (500 mg, 0.78 µmol), was phosphitylated with 2-cyanoethyl-N,N-diisopropyl-chlorophosphoramidite generally according to Song, *et al., Tetrahedron Lett.,* 1999, 40, 4153-4156. Following column chromatography with n-hexane/acetone/triethylamine (58/40/2) and precipitation in cold hexane, an amount of 550 mg (0.66 µmol, 85%) of the amidite was isolated. R1: (n-hexane/acetone/triethylamine 49/49/2) 0.38 LSIMS (positive mode, NPOE) 838 [M+H]⁺

### Solid phase oligonucleotide synthesis

Assembly was done at a 1 µmol scale (10 µmol for the Dickerson sequence) on a ABI 381A DN synthesizer following standard procedures such as those described by Song, *et al., Tetrahedron Lett.,* 1999, 40, 4153-4156, except for using a 0.12 M solution of the unnatural amidite with a three minute coupling time. Detritylation time for the monomethoxytrityl group was doubled. The ribooligonucleotides were assembled by Eurogentec.

### Mass spectrometry

The DNA 12-mer(5'-d(CGC GAA₁ TTC GCG-3')] was dissolved in water-acetonitrile (50:50) and desalted by means of Dowex-50W cation exchange beads. The supernatant was diluted by the addition of a tenfold volume of water- acetonitrile- ammonia (50:50:0.3%). Electrospray MS and MS/MS data were acquired on a Micromass Q-Tofmass spectrometer (Whytenshawe, Manchester, UK) fitted with a nanospray ion source (ESI-MS) and operated in the negative mode. The instrument was calibrated with a two-point calibration using the singly charged ion of the monomer and dimer of raffinose.

### CD Experiments

CD spectra were measure with a Jasco 600 spectropolarimeter in thermostatically controlled 1 cm cuvettes connected with a Lauda R CS 6 bath. The oligomers were dissolved and analyzed in a solution of NaCl (0.1M) potassium phosphate buffer (20 mM), pH 7.5, EDTA 0.1 mM at 25 °C and at a concentration of 5 µM for each strand.

### RNase H Experiment

The oligonucleotide 5'-CmGmGmCmGmrUrUrUrUrUrUCmAmGmGmAm-3' was radiolabelled (³²P) at the 5'-end using T₄ polynucleotide kinase (Gibco BRL) and (**gamma-**³²P] ATP (4500 Ci/mmol, ICN) by standard procedures and purified on a NAP-5® column (Pharmacia). The radiolabelled oligonucleotide (2.5 pmol) and the complementary oligonucleotide (50 nmol) with deoxy-, cyclohexene or anhydrohexitol building blocks respectively, were incubated in a total volume of 50 µl containing 10 mM Tris-chloride, pH 7.5, 25 mM KCl and 0.5 mM MgCl₂ at 37 °C for 15 min, allowing the duplex to be formed. Cleavage reactions were started by that addition of 30 U RNase H (*E. Coli* ribonuclease H, Amersham) to the mixture. Control tubes received no enzyme. Aliquots were taken at appropriate time intervals, mixed with an equal volume of stop mix (EDTA 50 mM, xylene cyanol FF 0.1% and bromophenol blue 0.1 % in formamide 90%) and chilled on ice. Samples were analyzed by denaturing 20% PAGE containing urea (50%) with TBE buffer at 1000 V for 1.25 hours. Results were visualized by phosphorimaging (Packard Cyclone, Optiquant software).

### Enzymatic stability

The oligonucleotides (dA)₁₃-propanediol, (A₁)₁₃-propanediol and (A₂)₁₃₋propanediol were radiolabelled (³²P) at the 5'-end using T₄ polynucleotide kinase (Gibco BRL) and (**gamma-**³²P] ATP (4500 Ci/mmol, ICN) by standard procedures and purified on a NAP-5® column (Pharmacia). In order to test for a broad range of enzymatic activity, 20 pmol radiolabelled oligonucleotide was incubated in 20 µL of 50% fetal calf serum in PBS at 37°C. Aliquots were taken at appropriate time intervals, mixed with double volume of stop mix (EDTA 50 mM, xylene cyanol FF 0.1% and bromophenol blue 0.1% in 7M urea,) and chilled on ice. Samples were analyzed by denaturing 20% PAGE containing urea (50%) with TBE buffer at 1000 V for 1.25 hours. Results were visualized by phosphorimaging (Packard Cyclone, Optiquant software).

### UV mixing curve

Experiments were performed in a buffer containing 0.1 M NaCl, 0.02 M potassium phosphate pH 7.5 and 0.1 µM EDTA at 10 °C. The different samples were prepared by mixing 4 µM solutions of both strands, (A₁)₁₃ and dT₁₃ at the appropriate ratio. The samples were allowed to stabilize for 2 hours after which UV absorbance was measured with a Varian Cary 300 Bio spectrophotometer at 260 nm. The curve was obtained by means of linear regression.

### NMR sample preparation and NMR spectroscopy

NMR samples were prepared by dissolving the oligomer in 300 µ of NMR buffer which consisted of 0.1 M NaCl, 10mM KH₂PO₄, 10 µM EDTA, pH 7.5. The sample was lyophilized from 99.96% D2O. The sample was finally dissolved in 30 µl 99.96% D2O (or 90% H2O and 10% D2O exchangeable proton NMR experiments). The samples were annealed by heating to 80 °C followed by slow cooling. The total concentration of the duplex was 1.4 mM. NMR spectra were recorded on a Varian 500 unity spectrometer (operating at 499.505 MHz) using 3 mm triple resonance (ACP) probe. Quadrature detection was achieved by States-Haverkom hypercomplex mode as generally described by States, *et al., J. Magn. Res.,* 1982, 48, 286-292. Spectra were processed using the programs NMRPipe (Delaglio, *et al., J. Biomol. NMR* 1995,6,277-293) and XEASY (Xia, *et al., Institute of Molecular Biology and Biophysics* 1994, Zürich, Switzerland) running on a Silicon Graphics O2 workstation (IRIX version 6.5). Exchangeable proton NMR spectra of the sample dissovled in 90% H₂O/10%D₂O were recorded at 20 °C using WATERGATE pulse sequence. Sweep widths of 12000 Hz in both dimensions were used with 32 scans, 2048 data points in *t*2 and 600 FIDs in *t*1. The data were apodized with a shifted sine-bell square function in both dimensions and processed to a 4K x 2K matrix. The 2D DQF-COSY, TOCSY and NOESY spectra from the sample in D₂O were recorded at 20 °C with sweep widths of 5000 MHz in both dimensions. The residual HDO peak was suppressed by presaturation. The ³¹P decoupled (on resonance continuous decoupling) DQF-COSY spectrum consisted of 4096 data points in *t*2 and 512 increments in *t*1. The data were apodized with a shifted sine-bell square function in both dimensions and processed to a 4K x 2K matrix. For the TOCSY experiment, a Clean MLEV 17 version was used with a TOCSY mixing time of 65 ms. The spectrum was acquired with 32 scans, 2048 data points in *t*2 and 512 FIDS in *t*1. The data were apodized with a shifted sine-bell square function in both dimensions and processed to a 4K x 2K matrix. The NOSEY experiments were acquired with mixing times of 50, 100, 150, 250 and 32 scans, 2048 data points in *t*2 and 512 increments in *t*1. A ¹H-³¹P HETCOR was acquired with 128 scans, 2048 datapoints in the proton dimension, *t*2, and 512 real data points in the phosphorus dimension, *t*1, over sweep widths of 5000 Hz and 2000 Hz, respectively.

## Claims

1. A compound comprising a plurality of covalently-bound units that individually include a nucleosidic base and a furanosyl moiety or a cyclohexenyl moiety covalently bound to said base, provided that said compound comprises at least one unit including a cyclohexenyl moiety.

2. The compound of claim 1, wherein the cyclohexenyl moiety is covalently bound to an adenine base.

3. The compound of claim 1 or 2, wherein said units are covalently bound such that the base portions thereof form a mixed base sequence that is complementary to a RNA base sequence or a DNA base sequence.

4. The compound of any one of claims 1 to 3, wherein at least one of said furanosyl moieties bears a 2'-hydroxyl group.

5. The compound of any one of claims 1 to 4, wherein a phosphodiester linkage or phosphorothioate linkage binds at least two of said units.

6. The compound of any one of claims 1 to 5, which induces RNase H activity.

7. A compound according to anyone of claims 1 to 6, for use as a medicament.

8. The compound of claim 7, wherein said medicament is for treatment of a disease associated with undesired protein production.

9. The compound of claim 7 or 8, which induces a reduction in protein production.

10. A pharmaceutical composition comprising a compound according to any one of claims 1 to 6 and a pharmaceutically acceptable carrier or diluent.

11. The pharmaceutical composition of claim 10, wherein said composition is in the form of a liquid.

12. The pharmaceutical composition of claim 10, wherein said composition is in the form of a powder.

13. A method comprising contacting a compound according to any one of claims 1 to 6 with RNA or DNA in vitro.

14. Use of the compound of any one of claims 1 to 6 in a non-therapeutic, non-diagnostic method comprising contacting the compound with RNA or DNA in vivo.

## Patentansprüche

1. Verbindung mit einer Vielzahl kovalent verbundener Einheiten, die individuell eine Nukleosidbase und einen an die Base kovalent gebundenen Furanosylrest oder Cyklohexenylrest enthalten, vorausgesetzt, dass die Verbindung zumindest eine einen Cyklohexenylrest enthaltende Einheit aufweist.

2. Verbindung nach Anspruch 1, wobei der Cyklohexenylrest kovalent an eine Adeninbase gebunden ist.

3. Verbindung nach Anspruch 1 oder 2, wobei die Einheiten kovalent so gebunden sind, dass ihre Basenanteile eine Mischbasensequenz bilden, die zu einer RNA-Basensequenz oder einer DNA-Basensequenz komplementär ist.

4. Verbindung nach einem der Ansprüche 1 bis 3, wobei zumindest einer der Furanosylreste eine 2'-Hydroxylgruppe trägt.

5. Verbindung nach einem der Ansprüche 1 bis 4, wobei eine Phosphodiesterbindung oder eine Phosphorthioatbindung zumindest zwei der Einheiten bindet.

6. Verbindung nach einem der Ansprüche 1 bis 5, die eine RNase H-Aktivität induziert.

7. Verbindung nach einem der Ansprüche 1 bis 6 zur Verwendung als Medikament.

8. Verbindung nach Anspruch 7, wobei das Medikament zur Behandlung einer mit unerwünschter Proteinproduktion assoziierten Erkrankung ausgelegt ist.

9. Verbindung nach Anspruch 7 oder 8, die eine Verringerung in der Proteinproduktion induziert.

10. Pharmazeutische Zusammensetzung, umfassend eine Verbindung nach einem der Ansprüche 1 bis 6 und einen pharmazeutisch verträglichen Träger oder ein pharmazeutisch verträgliches Verdünnungsmittel.

11. Pharmazeutische Zusammensetzung nach Anspruch 10, wobei die Zusammensetzung in Form einer Flüssigkeit vorliegt.

12. Pharmazeutische Zusammensetzung nach Anspruch 10, wobei die Zusammensetzung in Form eines Pulvers vorliegt.

13. Verfahren, umfassend das In-Kontakt-Bringen einer Verbindung nach einem der Ansprüche 1 bis 6 mit RNA oder DNA *in vitro.*

14. Verwendung der Verbindung nach einem der Ansprüche 1 bis 6 in einem nichttherapeutischen, nicht-diagnostischen Verfahren, umfassend das In-Kontakt-Bringen der Verbindung mit RNA oder DNA *in vivo.*

## Revendications

1. Composé comprenant une pluralité d'unités liées de façon covalente, qui incluent individuellement une base nucléosidique et une entité furanosyle ou une entité cyclohexényle liée de façon covalente à ladite base, à la condition que ledit composé comprenne au moins une unité incluant une entité cyclohexényle.

2. Composé selon la revendication 1, dans lequel l'entité cyclohexényle est liée de façon covalente à une base adénine.

3. Composé selon la revendication 1 ou 2, dans lequel lesdites unités sont liées de façon covalente de telle sorte que les parties bases de celles-ci forment une séquence de bases mélangées qui est complémentaire d'une séquence de bases d'ARN ou d'une séquence de bases d'ADN.

4. Composé selon l'une quelconque des revendications 1 à 3, dans lequel au moins une desdites entités furanosyle porte un groupe 2'-hydroxyle.

5. Composé selon l'une quelconque des revendications 1 à 4, dans lequel une liaison phosphodiester ou une liaison phosphorothioate relie au moins deux desdites unités.

6. Composé selon l'une quelconque des revendications 1 à 5, qui induit une activité RNase H.

7. Composé selon l'une quelconque des revendications 1 à 6, pour une utilisation en tant que médicament.

8. Composé selon la revendication 7, dans lequel ledit médicament est pour le traitement d'une maladie associée à la production indésirable de protéine.

9. Composé selon la revendication 7 ou 8, qui induit une diminution de la production de protéine.

10. Composition pharmaceutique comprenant un composé selon l'une quelconque des revendications 1 à 6 et un support ou un diluant pharmaceutiquement acceptable.

11. Composition pharmaceutique selon la revendication 10, dans laquelle ladite composition est sous la forme d'un liquide.

12. Composition pharmaceutique selon la revendication 10, dans laquelle ladite composition est sous la forme d'une poudre.

13. Procédé comprenant la mise en contact d'un composé selon l'une quelconque des revendications 1 à 6 avec de l'ARN ou de l'ADN in vitro.

14. Utilisation du composé selon l'une quelconque des revendications 1 à 6 dans un procédé non thérapeutique, non diagnostique, comprenant la mise en contact du composé avec de l'ARN ou de l'ADN in vivo.
